Europäisches Patentamt

European Patent Office

Office européen des brevets

(19)

(11) Numéro de publication : **0 101 127**
**B1**

(12) **FASCICULE DE BREVET EUROPÉEN**

(45) Date de publication du fascicule du brevet :
**02.01.86**

(51) Int. Cl.⁴ : **C 01 B   7/07**

(21) Numéro de dépôt : **83201155.5**

(22) Date de dépôt : **04.08.83**

(54) **Procédé pour l'obtention de chlorure d'hydrogène exempt d'acétylène à partir de mélanges contenant du chlorure d'hydrogène, du chlorure de vinyle et de l'acétylène.**

(30) Priorité : **13.08.82 FR 8214194**

(43) Date de publication de la demande :
**22.02.84 Bulletin 84/08**

(45) Mention de la délivrance du brevet :
**02.01.86 Bulletin 86/01**

(84) Etats contractants désignés :
**AT BE CH DE FR GB IT LI LU NL SE**

(56) Documents cités :
**DE-A- 1 568 679**
**DE-A- 2 353 437**
**DE-A- 2 438 153**
**US-H-   315 397**

(73) Titulaire : **SOLVAY & Cie (Société Anonyme)**
**Rue du Prince Albert, 33**
**B-1050 Bruxelles (BE)**

(72) Inventeur : **Closon, André**
**Rue du Champ St. Nicolas, 10**
**B-1488 Genappe (Bousval) (BE)**

Jouve, 18, rue St-Denis, 75001 Paris, France

Description

La présente invention concerne un procédé pour l'obtention de chlorure d'hydrogène exempt d'acétylène à partir de mélanges contenant du chlorure d'hydrogène, du chlorure de vinyle et de l'acétylène, notamment ceux provenant de la pyrolyse du 1,2-dichloréthane.

Lors de la fabrication du chlorure de vinyle par pyrolyse du 1,2-dichloréthane, il se forme des quantités importantes de chlorure d'hydrogène et de faibles quantités d'acétylène. Le chlorure de vinyle est habituellement séparé par une distillation à basse température avec obtention, en pied de colonne, notamment de chlorure de vinyle liquide et, en tête de colonne, de chlorure d'hydrogène contenant l'acétylène. Le chlorure d'hydrogène obtenu est généralement recyclé à la fabrication du 1,2-dichloréthane par oxychloration de l'éthylène. Le recyclage de chlorure d'hydrogène contenant de l'acétylène engendre la formation de produits polychlorés tels que des polychloréthylènes et des polychloréthanes. Ces produits sont des contaminants du 1,2-dichloréthane qui affectent le bon fonctionnement de l'installation de pyrolyse et la pureté du chlorure de vinyle obtenu.

Dès lors, différents procédés ont été proposés pour éliminer l'acétylène du chlorure d'hydrogène obtenu par pyrolyse du 1,2-dichloréthane.

Ainsi, dans le brevet CA-A-814 902 (THE DOW CHEMICAL COMPANY), on décrit l'élimination de l'acétylène et d'autres impuretés organiques du chlorure d'hydrogène par une réaction d'hydrochloration de ces impuretés en présence de trichlorure d'aluminium comme catalyseur. Les produits résultant de cette hydrochloration sont ensuite séparés du chlorure d'hydrogène par des moyens conventionnels tels que l'adsorption sélective sur une colonne de charbon actif ou la distillation à basse température.

Par ailleurs, le brevet GB-A-1 405 714 (IMPE-RIAL CHEMICAL INDUSTRIES LIMITED) se rapporte à une opération similaire mais où l'on fait intervenir des catalyseurs à base de cuivre ou de mercure tels que du chlorure mercurique déposé sur du charbon actif. Les produits de l'hydrochloration peuvent être séparés du chlorure d'hydrogène par des techniques conventionnelles telles que la distillation fractionnée.

Ces procédés connus présentent l'inconvénient de faire intervenir des opérations distinctes d'hydrochloration et de séparation, ce qui nécessite la mise en œuvre d'appareillages spécifiques et des dépenses énergétiques considérables. Par ailleurs, les quantités d'acétylène à éliminer sont tellement faibles qu'en pratique les produits de l'hydrochloration de l'acétylène ne sont pas valorisés.

La présente invention a pour but de fournir un procédé de ce genre qui ne présente plus les inconvénients précités. Le procédé selon l'invention nécessite peu d'appareillage spécifique et augmente le rendement global en chlorure de vinyle.

L'invention concerne un procédé pour l'obtention de chlorure d'hydrogène exempt d'acétylène à partir de mélanges contenant du chlorure d'hydrogène, du chlorure de vinyle et de l'acétylène, dans lequel on convertit l'acétylène en chlorure de vinyle à l'intervention d'un catalyseur d'hydrochloration et dans lequel on soumet le mélange à une distillation dans une colonne, on prélève dans la partie supérieure de la colonne une phase gazeuse constituée substantiellement de chlorure d'hydrogène et contenant l'acétylène, on met la phase gazeuse en contact avec le catalyseur d'hydrochloration, et on réintroduit ensuite la phase gazeuse dans la colonne.

Les mélanges contenant du chlorure de vinyle, du chlorure d'hydrogène et de l'acétylène soumis à la distillation peuvent provenir de la pyrolyse du 1,2-dichloréthane. Ils contiennent en général au moins 15 % molaires de chlorure de vinyle, 15 % molaires de chlorure d'hydrogène et moins de 1 % molaire d'acétylène. De préférence on applique le procédé selon l'invention à des mélanges contenant au moins 20 % molaires de chlorure de vinyle, 20 % molaires de chlorure d'hydrogène et moins de 0,5 % molaire d'acétylène. Les mélanges peuvent contenir en outre du 1,2-dichloréthane.

Selon l'invention, on soumet le mélange contenant du chlorure de vinyle, du chlorure d'hydrogène et de l'acétylène à une distillation dans une colonne qui permet de séparer le chlorure d'hydrogène récupéré en tête de colonne, du chlorure de vinyle, récupéré en pied. On peut utiliser à cet effet n'importe quelle colonne de distillation habituellement employée pour séparer le chlorure d'hydrogène et le chlorure de vinyle, et notamment des colonnes à plateaux ou à empilage. Lorsqu'on met en œuvre des mélanges ayant des compositions telles que précisées ci-avant, on utilise en général des colonnes comprenant au moins 25 plateaux théoriques ; la température dans le bouilleur est en général comprise entre 80 et 110 °C et la colonne fonctionne sous une pression comprise entre 8 et 12 bars. En général, le liquide récolté en pied de colonne contient au moins 30 % molaires de chlorure de vinyle. La phase gazeuse récupérée en tête de colonne contient au moins 99 % molaires de chlorure d'hydrogène. Ce chlorure d'hydrogène peut être recyclé directement à la fabrication du 1,2-dichloréthane par oxychloration de l'éthylène.

Selon l'invention, on prélève dans la partie supérieure de la colonne une phase gazeuse constituée, en général, d'au moins 95 % molaires de chlorure d'hydrogène. On choisit le niveau auquel le prélèvement est opéré dans la colonne en fonction de la composition souhaitée pour la phase gazeuse. De préférence, le prélèvement est opéré à un niveau tel que la phase gazeuse contient au moins 98 % molaires de chlorure d'hydrogène et au plus 0,1 % molaire de chlorure de vinyle. Les résultats les plus avantageux sont obtenus lorsque la phase gazeuse contient au moins 99 % molaires de chlorure d'hydrogène et au plus 0,01 % molaire de chlorure de vinyle. En général, la phase gazeuse contient moins de 2 % molaire d'acétylène et, le plus souvent, moins de 1 % molaire. En général, le prélèvement de la phase gazeuse est opéré dans le tiers supérieur de la colonne et, le plus souvent, à un niveau situé entre les deux-tiers et les trois-quarts de la hauteur de la colonne.

Selon un mode de réalisation préféré de l'invention, on prélève au niveau du prélèvement la totalité de la phase gazeuse montant dans la colonne. A cet effet, on obture totalement la colonne juste au-dessus du niveau du prélèvement, on recueille le liquide descendant au-dessus du niveau de l'obturation et on le réintroduit dans la colonne en dessous du niveau de l'obturation. De préférence, le liquide descendant est prélevé juste au-dessus du niveau de l'obturation et réintroduit juste en dessous de ce niveau.

Selon l'invention, la phase gazeuse prélevée est mise en contact avec un catalyseur d'hydrochloration. En général, on utilise à cet effet plusieurs réacteurs disposés en parallèle, de façon à pouvoir en mettre certains hors de service en vue d'assurer leur entretien. Les catalyseurs utilisés habituellement se trouvent sous la forme de particules solides. Dès lors, les réacteurs mis en œuvre sont généralement des réacteurs à lit fixe.

Comme catalyseur, on peut utiliser tous les catalyseurs d'hydrochloration de l'acétylène. De bons résultats ont été obtenus avec des sels de mercure, et plus particulièrement avec le chlorure mercurique, déposés sur charbon actif. Dans ces catalyseurs, la quantité de mercure est en général comprise entre 1 et 25 % en poids de la quantité de charbon actif. Les meilleurs résultats ont été obtenus avec des catalyseurs contenant entre 3 et 10 % en poids de mercure.

La température à laquelle la réaction est opérée peut varier entre de larges limites. Lorsque l'on met en œuvre un catalyseur constitué de chlorure mercurique déposé sur charbon actif à raison de 5 % en poids de mercure, la température est maintenue entre 75° et 150 °C. Le temps de séjour et, par conséquent, la quantité de catalyseur mise en œuvre sont choisis en fonction de la quantité maximum d'acétylène que l'on peut tolérer dans le chlorure d'hydrogène obtenu en tête de colonne de distillation.

De préférence la phase gazeuse est ensuite réintroduite dans la colonne de distillation à un niveau permettant une séparation optimale entre le chlorure d'hydrogène et le chlorure de vinyle présent à ce niveau. Selon le mode de réalisation préféré de l'invention, la phase gazeuse est réintroduite à un niveau supérieur à celui de l'obturation de la colonne. On obtient ainsi, en tête de colonne, du chlorure d'hydrogène contenant moins de 0,05 % molaire d'acétylène. De préférence, la phase gazeuse venant de l'hydrochloration est refroidie avant d'être réintroduite dans la colonne.

Le procédé selon l'invention peut être réalisé en continu ou en semi-continu.

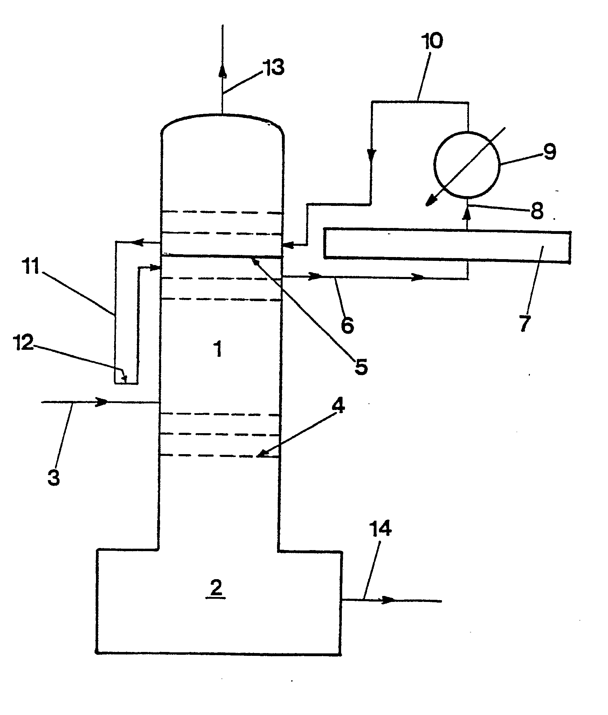

Un appareillage utilisable pour réaliser en continu le procédé selon l'invention est illustré schématiquement dans la figure unique du dessin annexé.

L'appareillage illustré comprend une colonne de distillation, pourvue d'un corps de colonne 1 et d'un bouilleur 2 contenant une phase liquide. Le corps de colonne 1 comprend une alimentation 3 pour l'introduction du mélange.

Le corps de colonne 1 comprend une série de trente-cinq plateaux 4 espacés régulièrement tout au long de la colonne ainsi qu'une obturation 5 située au vingt-cinquième plateau. Une voie 6 de prélèvement est aménagée, immédiatement en dessous de l'obturation de manière à amener la phase gazeuse à traverser le réacteur d'hydrochloration 7 contenant le catalyseur et doté d'un système de chauffage non représenté permettant de chauffer la phase gazeuse. Par la voie 8, on achemine la phase gazeuse vers l'échangeur de chaleur 9. La voie 10 sert à réintroduire la phase gazeuse dans la colonne juste au-dessus de l'obturation 5. La voie 11 achemine la phase liquide se trouvant au-dessus de l'obturation 5 vers un siphon 12 et le corps de colonne 1 à un niveau inférieur à celui de l'obturation 5. Par la voie 13, le chlorure d'hydrogène exempt d'acétylène quitte l'installation. La voie 14 sert à recueillir les composants du pied liquide.

L'invention est illustrée par l'exemple de réalisation décrit ci-après.

Exemple

On utilise une colonne de distillation telle que schématisée à la figure dont le diamètre est de 10 cm et comportant une série de trente-cinq plateaux. On utilise un réacteur ayant une capacité utile en catalyseur de 4,5 kg. Le catalyseur est constitué de charbon actif sur lequel on a déposé du chlorure mercurique à raison de 5 % en poids de mercure par kg de support.

On alimente la colonne avec un mélange contenant du chlorure de vinyle et du chlorure d'hydrogène à raison de 30 kg par heure de chlorure de vinyle et de 15 kg par heure de chlorure d'hydrogène. La pression à l'entrée du réacteur d'hydrochloration situé au vingt-cinquième plateau est de 9,9 bars effectifs et la température de 8 °C. La composition de la phase gazeuse prélevée dans la colonne par la voie 6 est de 99,6 % en volume de chlorure d'hydrogène, 0,38 % en volume d'acétylène et 0,003 % en volume de chlorure de vinyle.

Après passage dans le réacteur, chauffé à 105 °C, après un temps de contact d'environ 12 sec, la phase gazeuse comprend 99,6 % en volume de chlorure d'hydrogène, 0,37 % en volume de chlorure de vinyle et 0,006 % en volume d'acétylène. Par ailleurs, le chlorure d'hydrogène sortant en tête de colonne est d'une pureté de 99,9 % en volume et ne contient que 0,006 % en volume d'acétylène et quasi plus de chlorure de vinyle.

**Revendications**

1. Procédé pour l'obtention de chlorure d'hydrogène exempt d'acétylène à partir de mélanges contenant du chlorure d'hydrogène, du chlorure de vinyle et de l'acétylène, dans lequel on convertit l'acétylène en chlorure de vinyle à l'intervention d'un catalyseur d'hydrochloration, caractérisé en ce qu'on soumet le mélange à une distillation dans une colonne, on prélève dans la partie supérieure de la colonne une phase gazeuse constituée substantiellement de chlorure d'hydrogène et contenant l'acétylène, on met la phase gazeuse en contact avec le catalyseur d'hydrochloration, et on réintroduit ensuite la phase gazeuse dans la colonne.

2. Procédé selon la revendication 1, caractérisé en ce que l'on prélève la phase gazeuse à un niveau tel qu'elle contient au moins 98 % molaires de chlorure d'hydrogène et au plus 0,1 % molaire de chlorure de vinyle.

3. Procédé selon la revendication 2, caractérisé en ce que l'on prélève la phase gazeuse à un niveau tel qu'elle contient au moins 99 % molaires de chlorure d'hydrogène et au plus 0,01 % de chlorure de vinyle.

4. Procédé selon l'une quelconque des revendications 1 à 3, caractérisé en ce que l'on prélève la phase gazeuse à un niveau situé dans le tiers supérieur de la colonne.

5. Procédé selon l'une quelconque des revendications 1 à 4, caractérisé en ce que l'on prélève au niveau du prélèvement la totalité de la phase gazeuse montant dans la colonne.

6. Procédé selon la revendication 5, caractérisé en ce que l'on obture la colonne juste au-dessus du niveau du prélèvement, on recueille le liquide descendant au-dessus du niveau de l'obturation, et on le réintroduit dans la colonne au-dessous du niveau de l'obturation.

7. Procédé selon la revendication 6, caractérisé en ce que le liquide descendant est réintroduit dans la colonne juste en dessous du niveau d'obturation.

8. Procédé selon l'une quelconque des revendications 6 ou 7 caractérisé en ce que l'on réintroduit la phase gazeuse dans la colonne à un niveau supérieur à celui de l'obturation.

9. Procédé selon l'une quelconque des revendications 1 à 8, caractérisé en ce que l'on refroidit la phase gazeuse avant de la réintroduire dans la colonne.

10. Procédé selon l'une quelconque des revendications 1 à 9, caractérisé en ce que l'on utilise comme catalyseur d'hydrochloration du chlorure mercurique déposé sur du charbon actif.

## Claims

1. Process for obtaining hydrogen chloride free from acetylene from mixtures containing hydrogen chloride, vinyl chloride and acetylene, in which the acetylene is converted to vinyl chloride with the aid of a hydrochlorination catalyst, characterised in that the mixture is subjected to a distillation in a column, a gas phase consisting substantially of hydrogen chloride and containing acetylene is taken off in the upper part of the column, the gas phase is brought into contact with the hydrochlorination catalyst and then the gas phase is reintroduced into the column.

2. Process according to Claim 1, characterised in that the gas phase is taken off at level such that it contains at least 98 mol % of hydrogen chloride and at most 0.1 mol % of vinyl chloride.

3. Process according to Claim 2, characterised in that the gas phase is taken off at a level such that it contains at least 99 mol % of hydrogen chloride and at most 0.01 % of vinyl chloride.

4. Process according to any one of Claims 1 to 3, characterised in that the gas phase is taken off at a level located in the upper one-third of the column.

5. Process according to any one of Claims 1 to 4, characterised in that the whole of the gas phase rising in the column is taken off at the take-off level.

6. Process according to Claim 5, characterised in that the column is blocked just above the take-off level and the descending liquid is collected above the blocking level and is reintroduced into the column below the blocking level.

7. Process according to Claim 6, characterised in that the descending liquid is reintroduced into the column just below the blocking level.

8. Process according to any one of Claims 6 or 7, characterised in that the gas phase is reintroduced into the column at a level above the blocking level.

9. Process according to any one of Claims 1 to 8, characterised in that the gas phase is cooled before reintroducing it into the column.

10. Process according to any one of Claims 1 to 9, characterised in that mercuric chloride deposited on active charcoal is used as the hydrochlorination catalyst.

## Patentansprüche

1. Verfahren zur Erzeugung von acetylenfreiem Chlorwasserstoff, ausgehend von Chlorwasserstoff, Vinylchlorid und Acetylen enthaltenden Mischungen, wobei man das Acetylen zu Vinylchlorid unter Einwirkung eines Hydrochlorierungs-Katalysators umsetzt, dadurch gekennzeichnet, daß man die Mischung einer Destillation in einer Kolonne unterwirft, am oberen Teil der Kolonne eine Gasphase, die im wesentlichen aus Chlorwasserstoff besteht und Acetylen enthält, entnimmt, die Gasphase in Kontakt mit dem Hydrochlorierungs-Katalysator bringt und anschließend die Gasphase in die Kolonne zurückführt.

2. Verfahren nach Anspruch 1, dadurch gekennzeichnet, daß man die Gasphase in einer solchen Höhe entnimmt, daß sie mindestens 98 Mol-% Chlorwasserstoff und höchstens 0,1 Mol-% Vinylchlorid enthält.

3. Verfahren nach Anspruch 2, dadurch gekennzeichnet, daß man die Gasphase in einer solchen Höhe entnimmt, daß sie mindestens 99 Mol-% Chlorwasserstoff und höchstens 0.01 Mol-% Vinylchlorid enthält.

4. Verfahren nach einem der Ansprüche 1 bis 3, dadurch gekennzeichnet, daß man die Gasphase in einer Höhe, die sich im oberen Drittel der Kolonne befindet, entnimmt.

5. Verfahren nach einem der Ansprüche 1 bis 4, dadurch gekennzeichnet, daß man die gesamte in der Kolonne aufsteigende Gasphase auf der Entnahmehöhe entnimmt.

6. Verfahren nach Anspruch 5, dadurch gekennzeichnet, daß man die Kolonne knapp oberhalb der Entnahmehöhe verschließt, die herabfließende Flüssigkeit oberhalb der Verschlußhöhe sammelt, und diese unterhalb der Verschlußhöhe in die Kolonne zurückführt.

7. Verfahren nach Anspruch 6, dadurch gekennzeichnet, daß die herabfließende Flüssigkeit knapp unterhalb der Verschlußhöhe in die Kolonne zurückgeführt wird.

8. Verfahren nach einem der Ansprüche 6 oder 7, dadurch gekennzeichnet, daß man die Gasphase in einer Höhe, die oberhalb derjenigen des Verschlusses ist, in die Kolonne zurückführt.

9. Verfahren nach einem der Ansprüche 1 bis 8, dadurch gekennzeichnet, daß man die Gasphase abkühlt bevor man sie in die Kolonne zurückführt.

10. Verfahren nach einem der Ansprüche 1 bis 9, dadurch gekennzeichnet, daß man als Hydrochlorierungs-Katalysator Quecksilber-II-Chlorid auf Aktivkohleträger verwendet.